Europäisches Patentamt

European Patent Office

Office européen des brevets

(11) Publication number: **0 305 564**
**A1**

# EUROPEAN PATENT APPLICATION

(21) Application number: **87112754.4**

(22) Date of filing: **01.09.87**

(51) Int. Cl.⁴: **C07C 85/18**

(43) Date of publication of application:
**08.03.89 Bulletin 89/10**

(84) Designated Contracting States:
**CH DE FR GB IT LI NL**

(71) Applicant: **AIR PRODUCTS AND CHEMICALS, INC.**
**Route no. 222**
**Trexlertown Pennsylvania 18087(US)**

(72) Inventor: **Deeba, Michel**
**c/o Englehard Corp. Menlo Park**
**Edison, NJ 08818(US)**

(74) Representative: **Dipl.-Ing. Schwabe, Dr. Dr.**
**Sandmair, Dr. Marx**
**Stuntzstrasse 16**
**D-8000 München 80(DE)**

(54) **Amines via the amination of olefins using dealuminated zeolites.**

(57) This invention pertains to an improved catalytic process for the amination of $C_{2-8}$ olefins, the catalyst being a dealuminized zeolite having an increased Si:Al molar ratio over original zeolite. The dealuminized H-mordenite catalyst is particularly effective for the amination of isobutylene to give tert-butylamine.

EP 0 305 564 A1

# AMINES VIA THE AMINATION OF OLEFINS USING DEALUMINATED ZEOLITES

## TECHNICAL FIELD

This invention relates to a catalytic process for producing amines by the reaction of ammonia or ammonia type compound with an olefin over partially dealuminated zeolites.

## BACKGROUND OF THE PRIOR ART

The earliest work relating to the manufacture of amines by the amination of olefins particularly ethylamines by the amination of ethylene, appears to have been done by Teter et al as noted in U.S. Patents 3,623,061; 2,479,879; 2,417,892; 2,381,470; 2,658,041; 2,381,709; 2,392,107 and 2,398,899. These patents show that ammonia can be made to react with olefins, e.g. ethylene to produce amines. As noted by others through improvements and polyolefins were produced by the Teter et al. catalyst which is a metal supported on a spinel type support, silica and diatomaceous earth and the like.

Olin et al in U.S. Patents 2,422,631 and 2,422,632, discloses a process for producing amines and amides by the reaction of a monounsaturated olefin, carbon monoxide and an amine or ammonia. The catalyst used is a combination of a dehydrating and hydrogenation catalyst, e.g. nickel and activated alumina, copper and silica gel, etc.

Whitman, U.S. 2,501,556 dicloses a liquid phase reaction of ammonia and ethylene in the presence of an alkali metal catalyst to form ethylamine.

McClain, U.S. 3,412,158 discloses a gas phase process for producing primary alkyl amines from low molecular weight olefins and ammonia by carrying out the gas phase process in the presence of a noble metal containing catalyst at temperatures of from 90-175°C, at pressures of from atmospheric to 2,000 psig.

Peterson, et al. in U.S. 4,307,250 and 3,375,002 disclose the catalytic amination of a $C_{2-8}$ olefin using a synthetic crystalline alumino-silicate or a naturally occurring zeolite as a catalyst. Various olefins, such as, ethylene, propylene and isobutylene are reacted with ammonia or an amine in the presence of the zeolitic catalyst to produce the amine product.

## SUMMARY OF THE INVENTION

This invention relates to an improved process for the preparation of amines, particularly a branched $C_4$ olefin, by the vapor phase catalytic reaction of an olefin having from 2 to 8 carbon atoms and ammonia or a primary or secondary amine. In the basic process the olefin is reacted at a temperature and pressure sufficient to effect formation of the amine, but controlled to prevent polymer formation. The improvement generally resides in the use of a partially dealuminated crystalline alumino-silicate as the catalyst.

There are several advantages-associated with the invention as comparted to prior art processes. These advantages include:

greater catalytic activity than is achieved with non-dealuminated zeolite catalysts;

an ability to form a variety of alkyl amines, particularly $C_4$, at much higher rates of conversion without sacrificing the selectivity; and

outstanding catalyst life as in the case of Y zeolite presumably caused by improved zeolite stability.

## DETAILED DESCRIPTION OF THE INVENTION

Olefins having from 2 to 8 carbon atoms in the structure can be converted by vapor phase amination to the amine. The olefins can be mono or polyunsaturated, e.g. a diene. However, a monounsaturated olefin which is an alpha-beta unsaturated aliphatic olefin is preferred. Dienes, particularly conjugated dienes, have a tendency to polymerize and the temperature and pressure have to be closely monitored to prevent polymerization. Examples of olefins suited for producing amines by this process are ethylene, propylene, butylene, isobutylene, pentene, hexene, cyclohexene, cyclopentadiene and butadiene. Or these, the $C_4$ monoolefins are preferred in practicing the invention and these would include systems such as isobutylene.

In the amination of the olefins, an ammonia or an ammonia type compound, i.e. a primary or secondary amine is used as the co-reactant. If one uses a primary or secondary amine as the reactant, secondary and tertiary amines are formed as product. Suitable primary and secondary amines can be represented by the formulas $RNH_2$ and $(R)_2NH$ where each R is a hydrocarbon group having from 1-6 carbon atoms in the structure. Examples of amines include methylamine, dimethylamine, mono and di-n-propylamine, cyclohexylamine and the like. Lower alkyl amines are preferred.

In the process, an ammonia type compound is reacted with the olefin at temperatures broadly from about 150-500° C, but preferably at temperatures of about 200-400° C. Lower temperatures result in lower conversion and higher temperatures result in lower selectivity which generally appears in the form of polymer, nitrile formation or other by-products.

Pressures suited for practicing the invention are from about 300-6,000 psig with preferred pressures of from about 600-1100 psig. Generally, pressures lower than about 600 psig result in poor conversion of the olefin to amine. On the other hand, as the pressure is increased above about 1500 psig conversion is increased only slightly. However, selectivity to the amine decreases particularly when conjugated dienes, such as butadiene are used as the reactants. In order to minimize the recovery problems associated with separating the amine from the reactants and other by-products, higher conversions can be waived and pressures of from about 600-1100 psig used as this permits extremely high selectivities, e.g. greater than 95%.

Another important variable in the gas phase amination of the olefin is the mole ratio of ammonia to olefin in the feed. Generally, the mole ratio can be from about 0.2-20 moles ammonia per mole of olefin with preferred ranges of from about 1-10 to 1. Mole ratios higher than about 10:1 of course require greater energy expenditure to handle the larger quantities of gas that pass through the reactor, and there seems to be no significant advantage in terms of increased selectivity or conversion at these higher ratios of ammonia to olefin. On the other hand, as the molar ratio of ammonia to olefin falls below about 0.2, there is a loss of selectivity to the amine.

The gas hourly space velocity (GHSV) which has units of volume of gas per volume of catalyst in unit time i.e. (cc gas at STP)/(cc catalyst hours $^{-1}$). The gas hour space velocity can be from about 500-5,000 with a preferred range being from about 750-2,000. As the space velocity is increased toward the upper end of the range, conversion generally falls dramatically, particularly above about 3,000. Typically, conversions of from about 3-20% are achieved at space velocities of about 500-3,000.

The catalyst utilized in the amination process of this invention is commonly referred to as a partially dealuminated zeolite. As has been demonstrated by Peterson, et al. zeolitic materials, both natural and synthetic have been demonstrated to have catalytic capability for the amination of $C_2$ to $C_8$ olefins. Enhanced conversions are normally noted with those zeolitic materials which are in the acid form, e.g., the hydrogen ion or rare earth metal ion exchanged zeolites. Representative examples of zeolites suited for use in the acid form include Y, X, clinoptilolite, offretite, erionite, mordenite, montmorillonite and the ZSM family.

The dealumination of the zeolitic materials has been found to enhance the conversion of the olefin to amine without interfering with the selectivity of the catalyst system. Partial dealumination also improves catalyst life presumably because stability of the zeolite is improved by increasing the silica to alumina ratio. It also improves conversion presumably by reducing the resistance reactant diffusion within the zeolite pores. By dealumination it is meant that the zeolite is treated with a dealuminating agent such as a chelating agent or acid for the purpose of removing aluminum atoms from the zeolite structure. Typically the percent aluminum removed is sufficient to effect removal of 10-90% from the total alumina ($Al_2O_3$ by weight) present in the zeolite material. For example, mordenite normally has an Si to Al molar ratio of about 5:1. The Si to Al molar ratio for partially dealuminated mordenite should be from 6 to 100:1 with preferred molar ratios of 6 to 20:1. The silicon to aluminum molar ratio of a Y zeolite is about 2.2:1. The Si to Al ratio for a partially dealuminated Y zeolite should be from 2.5 to 50:1 with a preferred Si to Al molar ratio of 2.5 to 15:1.

There are several techniques available for removing aluminum atoms from the structure and some involve the treatment of the zeolite material with a chelating agent such as acetyl acetonate, ethylenediaminetetracetic acid, or by treatment with a mineral acid e.g. hydrochloric acid or by activation using silicon tetrachloride. Aluminum atom removal is effective at temperatures from about 25-100° C within a timeframe of 1 to 24 hours followed by calcination at temperatures of about 400°C. Examples of various techniques for removing aluminum atoms from zeolite materials to produce catalytic compositions are noted in U.S. 3,506,400; U.S. 3,937,791; and U.S. 3,761,396; these techniques are incorporated by reference.

The following examples are provided to illustrate various embodiments to the invention.

Example 1

Preparation of Dealuminated H-Y Zeolite.

A sodium Y zeolite sold under the designation LZ-Y52 by the Linde Division of Union Carbide Corporation was exchanged and partially dealuminated by the addition of 60-80 grams of 1/16″ extrudate to 2 liters of distilled water. Ammonium chloride was added to the distilled water to bring the level to 1-2 molar concentration. Hydrochloric acid (35% by weight) was added dropwise to bring the pH to a level of 3-5 and never less than a pH of 3. At this point the solution containing the zeolite was refluxed for 2 hours at fixed pH.

A dealuminated catalyst was recovered by washing the refluxed zeolite with hot distilled water and drying at 150°C. This procedure was repeated twice or more at 150°C to vary the degree of dealumination. The X-ray diffraction pattern of the partially dealuminated zeolite showed no significant loss in percent crystallinity. The Si to Al molar ratio was about 2.5:1 and higher.

Example 2

Preparation of Dealuminated H-Mordenite

A 60-80 gram sample of 1/16″ extrudate of H-mordenite sold by the Norton Company under designation Z-900H was dealuminated by adding this catalyst to 2 liters of distilled water containing from 5-50 milliters of 12 normal aqueous hydrochloric acid and 1 molar ammonium chloride. The solution was refluxed for 2 hours, washed with hot water until no chloride ions were detected and then dried at 150°C. The dealumination procedure was then repeated to achieve the desired degree of dealumination. The Si to Al molar ratio was about 6:1 and higher. The X-ray analysis of the dealuminated zeolite showed no loss of crystallinity.

Example 3

A series of amination runs were carried out by reacting ethylene, propylene, or isobutylene with ammonia in the presence of a variety of zeolitic catalysts in both the acid form and partially dealuminated form. The reactions were carried out in a tubular reactor having an internal diameter of 1/4 inch and included a thermal well of 1-1/4″ outside diameter axially extending from the bottom portion of the reactor. The reactor was filled with catalyst pellets as specified. Tables I-VIII provide reaction conditions, reactants and the results obtained. The Si to Al mole ratios are approximate. Unless specified otherwise the ammonia to olefin ratio (N/R) was 4:1 and the pressure was 750 psig. These conditions typically have been shown to be optimum for zeolite catalysts.

TABLE I

| EFFECT OF ACID TREATMENT ON % ETHYLENE CONVERSION OVER H-Y ZEOLITE | | | | | |
|---|---|---|---|---|---|
| Temp. °C | % Ethylene Conv. | | | $\frac{B}{A}$ | $\frac{C}{A}$ |
| | A | B | C | | |
| 320 | 1.1 | 1.5 | 1.6 | 1.36 | 1.45 |
| 340 | 3.0 | 3.9 | 3.8 | 1.3 | 1.27 |
| 360 | 5.1 | 7.8 | 7.9 | 1.5 | 1.55 |
| 380 | 9.9 | 12.3 | 13.5 | 1.24 | 1.36 |
| A = H-Y Zeolite (Control) | | | | | |
| B = Dealuminated H-Y Zeolite (once treated), Si:Al molar ratio of 2.5:1 | | | | | |
| C = Dealuminated H-Y Zeolite (twice treated) Si:Al molar ratio is Al 3:1 | | | | | |

Normally, the extrudate form of catalyst, whether treated or untreated, results in lower conversion levels e.g., about 1-2% than the non extruded form. However, the above results show that the dealuminated catalyst was superior to the nondealuminized control. Catalyst C shows about 40 to 50% improvement over Catalyst A with good results being achieved at the 360-380°C level. It also shows modest improvement over the once treated Y Zeolite which has slightly lower silicon to alumina ratio.

Aging studies were conducted on two H-Y zeolites prepared from Na-Y zeolite (Linde LZ-Y52. The control sample (sample A) maintained a constant 9.8% ethylene conversion of about 9.8% for five days at 365°C, N/R = 4, GHSV = 1000v/v/m, and then deactivated at 0.5% conversion per day. The catalyst had less than 5% ethylene conversion after ten days on stream. The X-ray diffraction pattern of the aged catalyst showed that the catalyst lost most of its crystallinity.

A second aging test was carried out on a partially dealuminated H-Y of the type described in Example 1 (Catalyst C). At 365°C, an N/R of 4 and GHSV 1000 hr⁻¹ (same conditions as above) the catalyst started at 10.5% ethylene conversion and deactivated at 0.14% per day for ten days. This is much slower than the deactivation rate of the control catalyst or any rare earth Y zeolite tested (a rare earth Zeolite deactivated at 0.25% per day). The X-ray diffraction pattern also showed no loss in crystallinity. The catalyst even retained crystallinity after aging tests at 375°C.

TABLE II

| EFFECT OF ACID TREATMENT ON ETHYLENE CONVERSION OVER H-MORDENITE | | | | | |
|---|---|---|---|---|---|
| Temp. °C | % Ethylene Conv. | | | $\frac{B}{A}$ | $\frac{C}{A}$ |
| | A | B | C | | |
| 320 | 0.4 | 0.8 | - | 2 | - |
| 340 | 1.8 | 2.4 | 2.9 | 1.3 | 1.67 |
| 360 | 4.5 | 6.3 | 6.1 | 1.4 | 1.35 |
| 370 | 6.3 | - | 8.45 | - | 1.3 |
| 380 | 7.8 | 10.3 | 11.5 | 1.3 | 1.5 |
| A = H-Mordenite (Z-900H) (control) Si to Al of 5:1 | | | | | |
| B = partially dealuminated H-Mordenite (Extracted once) Si/Al mole ratio 6:1 | | | | | |
| C = partially dealuminated H-Mordenite (Extracted twice) Si/Al mole ratio 8:1 | | | | | |

TABLE III

| EFFECT OF ACID TREATMENT ON ETHYLENE CONVERSION OVER H-CLINOPTILOLITE | | | | |
|---|---|---|---|---|
| Temp. °C | % Ethylene Conv. | | $\frac{B}{A}$ | Approximate % Improvement |
| | A | B | | |
| 320 | 1.9 | 2.45 | 1.3 | 30 |
| 340 | 4.6 | 5.6 | 1.2 | 20 |
| 360 | 7.9 | 10.9 | 1.4 | 40 |
| 370 | 10.1 | 11.9 | 1.2 | 20 |
| 380 | 12.0 | 14.2 | 1.2 | 20 |
| A = H-Clinoptilolite (Control) of Si:Al ratio of 5:1 B = Acid treated H-Clinoptilolite at a pH of 2.5 under reflux conditions: Si:Al ratio was 7:1 | | | | |

TABLE IV

| EFFECT OF PARTIAL DEALUMINATION ON PROPYLENE AMINATION ACTIVITY OVER LZ-Y82 | | | | | | |
|---|---|---|---|---|---|---|
| Temp. °C | GHSV | % Propylene Conv. | | | $\frac{B}{A}$ | $\frac{C}{A}$. |
| | | A | B | C | | |
| 320 | 2000 | 1.34 | 1.6 | 2.0 | 1.5 | 1.5 |
| 340 | 2000 | 3.5 | 4.0 | 4.2 | 1.14 | 1.2 |
| 350 | 2000 | 4.69 | 5.03 | 5.2 | 1.1 | 1.1 |
| 350 | 1000 | 5.8 | 6.1 | 6.3 | 1.05 | 1.1 |
| A = LZ-Y82 is a commercial H-Y Zeolite from Linde (control); it is an ultrastable Y zeolite. Si:Al of 1.8 B = HCL Treated LZ-Y82 Si:Al mole ratio of 2.5:1 C = EDTA Treated LZ-Y82 Si:Al mole ratio of 2.5:1 | | | | | | |

## TABLE V

### EFFECT OF H-Y AND ACID TREATED
### H-Y ZEOLITE ON PROPYLENE CONVERSION

| Temp.°C | GHSV | A LZ-Y62[a] | B LZ-Y72[a] | C H-Y[b] | D H-Y[c] | E H-Y[c] | F EDTA[d]/H-Y |
|---|---|---|---|---|---|---|---|
| | | | | % Propylene Conversion | | | |
| 300 | 2000 | 0.20 | 0.27 | 0.40 | 0.50 | 0.65 | 0.60 |
| 320 | 2000 | 0.90 | 0.90 | 1.62 | 1.82 | 1.93 | 2.0 |
| 340 | 2000 | 2.45 | 2.65 | 3.8 | 4.50 | 4.60 | 4.60 |
| 350 | 2000 | 3.45 | 3.7 | 5.0 | 5.27 | 5.2 | 5.8 |
| 350 | 1000 | 4.90 | 5.10 | 5.80 | 6.75 | 7.0 | 6.8 |

a) LZ-Y62 and LZ-Y72 (Catalysts A&B) are two commercial H-Y Zeolites from Linde. (Controls)

b) H-Y Zeolite (Catalyst C) is a partially dealuminated laboratory exchanged LZ-Y52 (Linde NaY) to the same level as LZ-Y62 and LZ-Y72. The pH of the solution during exchange was kept around 5. Si to AL mole ratio was 2.5:1

c) Partially dealuminated H-Y where some of the alumina was leached out to increase the Si to Al ratios. (Catalysts D and E). Si to Al ratio 3:1

d) A partially dealuminated H-Y Zeolite dealuminated with EDTA (Ethylenediaminetetracetic acid) (Catalyst F).

### TABLE VI

| COMPARISON OF CONVERSION RATIOS OF PARTIALLY DEALUMINATED H-Y ZEOLITE TO CATALYST B (LZ-Y72) TABLE V | | | | | |
|---|---|---|---|---|---|
| Temp.°C | GHSV | RATIOS | | | |
| | | $\frac{C}{B}$ | $\frac{D}{B}$ | $\frac{E}{B}$ | $\frac{F}{B}$ |
| 300 | 2000 | 1.5 | 1.9 | 2.4 | 2.2 |
| 320 | 2000 | 1.8 | 2.0 | 2.1 | 2.2 |
| 340 | 2000 | 1.4 | 1.7 | 1.7 | 1.7 |
| 350 | 2000 | 1.35 | 1.35 | 1.4 | 1.6 |
| 350 | 1000 | 1.14 | 1.3 | 1.4 | 1.35 |

TABLE VII

| EFFECT OF H-MORDENITE AND PARTIALLY DEALUMINATED H-MORDENITE ON PROPYLENE CONVERSION | | | | | | | |
|---|---|---|---|---|---|---|---|
| Temp.°C | % Propylene Conversion | | | | $\frac{B}{A}$ | $\frac{C}{A}$ | $\frac{D}{A}$ |
| | A | B | C | D | | | |
| | H-Mord.(Z-900H) | A.T.ᵃ/H.M. | A.T.ᵃ/H.M. | A.T.ᵃ/H.M. | | | |
| 300 | 0.62 | 1.16 | 1.3 | 1.3 | 1.9 | 2.1 | 2.1 |
| 320 | 2.0 | 3.4 | - | 4.0 | 1.7 | - | 2.0 |
| 340 | 4.1 | 5.2 | 5.3 | 6.5 | 1.26 | 1.3 | 1.6 |
| 350 | 4.35 | 5.6 | 5.9 | 7.1 | 1.3 | 1.36 | 1.6 |
| 350 (1000 hr-1) | 5.7 | 7.25 | - | - | 1.3 | - | - |
| The Si/Al mole ratio of H-mordenite was 5:1 and the acid treated H-mordenite Si/Al mole ratio was 8:1 (Catalyst C) and 12:1 (Catalyst D) | | | | | | | |

a) A.T. refers to acid treated. (partially dealuminated)

b) B/A, C/A and D/A are the conversion ratios achieved with partially dealuminated H-mordenite as compared to as received nondealuminated H-mordenite (Z-900H).

TABLE VIII

| COMPARISON BETWEEN COMMERCIAL ZEOLITES AND ACID TREATED H-MORDENITE FOR ISOBUTYLENE AMINATION | | | | | | | |
|---|---|---|---|---|---|---|---|
| Temp.°C | N/R | GHSV | A | B(H-Y) | C | $\frac{C}{A}$ | $\frac{C}{B}$ |
| | | | H-Mord. | LZ-y72 | A.W./H.M. | | |
| 220 | 2 | 1000 | 0.9 | 0.7 | 3.8 | 4.2 | 5.4 |
| 240 | 2 | 1000 | 2.9 | 3.3 | 10.1 | 3.5 | 3.1 |
| 260 | 2 | 1000 | 5.9 | 7.8 | 13.6 | 2.3 | 1.74 |
| A = H-Mordenite control Si/Al mole ratio was 5:1 | | | | | | | |
| B = a Commercial H-Y Zeolite from Linde having an Si/Al mole ratio of 2.5:1 | | | | | | | |
| C = Dealuminated H-mordenite the mole ratio Si/Al was 8:1 | | | | | | | |

These results show that the dealuminated mordenite was substantially more effective in aminating isobutylene to give tert butylamine than the control zeolite or any commercially available zeolite. Note the activity improved 3 to 5 times with the dealuminized zeolite and there was no loss in selectively. It was significantly better than any commercial catalyst (e.g. LZ-y72). The improvement in activity is less significant at 260°C and 280°C. presumably because of the thermodynamic limit of isobutylene conversion at 260°C of about 13%.

In summary, Tables I-VIII show the olefin conversion improved dramatically when the dealumined zeolite was used in place of the nondealuminzed zeolite under the same reaction conditions. The data also shows as the Si:Al mole ratio was increased by further treatment activity also increased. Compare Table V, VII and VIII where Si:Al mole ratios were at 2.5:1 and above. When isobutylene was aminated, the dealuminized zeolite improved conversion at temperatures from 220-260°C about 3 to 4 times over that obtained with the nondealuminized zeolites.

## Claims

1. In a vapor phase catalytic amination process for the production of amines by reacting a reaction mixture comprising an olefin having from 2-8 carbon atoms and a nitrogen compound selected from the group consisting of ammonia and primary and secondary amines at a temperature and pressure sufficient for effecting formation of said amine, said reaction being carried out in the presence of the crystalline alumina silicate as a catalyst, the improvement which comprises the utilization of a crystalline alumino-silicate which has been dealuminated.

2. The process of Claim 1 wherein said dealuminated crystalline alumino-silicate has sufficient aluminum atoms removed to reduce the weight of alumina 10-90% by weight of the total alumina in the zeolite.

3. The process of Claim 2 wherein said olefin is a $C_{2-4}$ olefin.

4. The process of Claim 3 where said crystalline alumino-silicate which has been dealuminated is a Y zeolite or mordenite.

5. The process of Claim 4 wherein said dealuminated Y zeolite or mordenite is ion exchanged with a trivalent rare earth metal ion or hydrogen ion.

6. The process of Claim 5 wherein the Si to Al mole ratio is from 6-100:1 for said mordenite and 2.5-15:1 for said Y zeolite.

7. The process of Claim 5 wherein the mole ratio of ammonia to olefin is from about 1-10:1.

8. The process of Claim 6 wherein said olefin is isobutylene.

9. The process of Claim 6 wherein said olefin is ethylene.

10. The process of Claim 8 wherein the pressure utilized in carrying out the amination of iso- butylene is from 600-1100 psig and the gas hourly space velocity is 750-2000 hr $\bar{s}^1$.

11. The process of Claim 8 wherein said amination of ethylene is carried out at a pressure in the range of 600-1100 psig and the gas hourly space velocity is from 750-2000 hr $\bar{s}^1$.

European Patent Office

# EUROPEAN SEARCH REPORT

Application Number

EP 87 11 2754

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 180 983 (AIR PRODUCTS AND CHEMICALS) * Claims; page 5 * | 1-11 | C 07 C 85/18 |
| D,Y | EP-A-0 039 918 (AIR PRODUCTS AND CHEMICALS) * Claims * | 1 | |
| D,Y | US-A-3 506 400 (P.E. EBERLY) * Claims * | 1 | |
| D,Y | US-A-3 640 681 (P.E. PICKERT) * Claims * | 1 | |

TECHNICAL FIELDS SEARCHED (Int. Cl.4)

C 07 C 85/00

The present search report has been drawn up for all claims

| Place of search | Date of completion of the search | Examiner |
|---|---|---|
| THE HAGUE | 03-05-1988 | MOREAU J.M. |